(19) Europäisches Patentamt
European Patent Office
Office européen des brevets

(11) **EP 3 731 735 B1**

(12) **EUROPEAN PATENT SPECIFICATION**

(45) Date of publication and mention of the grant of the patent:
**11.02.2026   Bulletin 2026/07**

(21) Application number: **18829835.0**

(22) Date of filing: **20.12.2018**

(51) International Patent Classification (IPC):
***A61B 5/00*** *(2006.01)*

(52) Cooperative Patent Classification (CPC):
**A61B 5/0077; A61B 5/442;** A61B 5/0002;
A61B 2562/046

(86) International application number:
**PCT/EP2018/086113**

(87) International publication number:
**WO 2019/129627 (04.07.2019 Gazette 2019/27)**

(54) **DEVICE FOR IMAGING SKIN**

VORRICHTUNG ZUR HAUTBILDGEBUNG

DISPOSITIF POUR IMAGERIE DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **27.12.2017   EP 17210613**

(43) Date of publication of application:
**04.11.2020   Bulletin 2020/45**

(73) Proprietor: **Koninklijke Philips N.V.**
**5656 AG Eindhoven (NL)**

(72) Inventors:
• **RAS, Arnoldus Johannes Martinus Jozeph**
**5656 AE Eindhoven (NL)**
• **HERMANS, Walter**
**5656 AE Eindhoven (NL)**

• **VARGHESE, Babu**
**5656 AE Eindhoven (NL)**

(74) Representative: **Philips Intellectual Property & Standards**
**High Tech Campus 52**
**5656 AG Eindhoven (NL)**

(56) References cited:
CN-U- 206 576 851          US-A1- 2014 243 685
US-A1- 2015 297 130          US-B2- 7 623 907

• DIMITRIOS KAPSOKALYVAS* A, NICOLA BRUSCINO B, DOMENICO ALFIERI C, VINCENZO DE GIORGI B, GIOVANNI CANNAROZZO B: "Imaging of human skin lesions with the Multispectral Dermoscope", SPIE, PO BOX 10 BELLINGHAM WA 98227-0010 USA, vol. 7548, 2010, XP040516411

**Description**

FIELD OF THE INVENTION

The invention relates to a device for imaging skin, a method of operating the device to image skin and a system for measuring skin texture.

BACKGROUND OF THE INVENTION

In the skincare field, it is valuable to acquire images of the skin. For example, images of the skin can be useful for monitoring the state of the skin (such as any deterioration or improvement in the state of the skin) and to detect any skin conditions.

Some existing devices for imaging skin comprise a light source for illuminating the skin while an image is acquired by an imaging sensor of the device. JP 2013/026846 discloses an example of a skin observation device where light is applied to the skin. This skin observation device uses a different angle of incidence of light with respect to the optical axis for observing skin texture to that used for observing skin spots.

US 2014/0243685 A1 discloses a dermatoscope having a generally circular viewing opening, a plurality of light sources including first and second groups of light sources arranged about the viewing opening, a first polarizer for polarizing light passing through the viewing opening, and a second polarizer for polarizing light emitted from the first group of light sources, wherein the first and second groups of light sources are arranged at different distances from a center of the viewing opening so that light from the second group of light sources is not polarized by the second polarizer. The angle of incidence of the polarized light relative to the skin surface is preferably within a range of approximately 45 to 55 degrees.

US 2015/0297130 A1 discloses a method to discriminate between benign and malignant tissue lesions using images.

**In** "Imaging of human skin lesions with the Multispectral Dermoscope" Kapsokalyvas et al. discuss a multispectral dermoscope employing high luminance LEDs with emission at three distinct spectral regions, wherein the illumination is polarized and an analyzer is used for detection.

However, while there exist such devices for imaging skin, the existing devices can suffer from poor image quality. For example, the images acquired by the existing devices are often not of a high enough contrast (or resolution) for features of the skin (such as skin texture, fine lines and wrinkles, skin spots, impurities, irregularities, abnormalities, etc.) to be resolved to an adequate degree.

There is thus a need for an improved device for imaging skin, which overcomes the existing problems.

SUMMARY OF THE INVENTION

As noted above, a limitation with existing devices is that the images acquired by such devices are often not of a high enough contrast (or resolution) for features of the skin (such as skin texture, fine lines and wrinkles, skin spots, impurities, irregularities, abnormalities, etc.) to be adequately resolved. It would thus be valuable to have an improved device for imaging skin, which overcomes the existing problems.

The invention is defined by the claims.

Therefore, according to a first aspect, there is provided a device comprising a light source configured to emit light to illuminate the skin. The light source has a radiation angle in a range from 5 to 40 degrees. The radiation angle is the angle at which an intensity of the light emitted by the light source is half that of a maximum intensity of the light emitted by the light source. The device also comprises an imaging sensor configured to detect light reflected from the skin and convert the detected light into an image of the skin.

In some embodiments, the light source may have a radiation angle in a range from 10 to 30 degrees. In some embodiments, the light source may have a radiation angle in a range from 10 to 25 degrees.

The light source is configured to emit light with an angle of incidence in a range from 10 to 30 degrees. In some embodiments, the light source may be configured to emit light with an angle of incidence in a range from 10 to 25 degrees.

In some embodiments, the light source may be configured to emit light to uniformly illuminate the skin. In some embodiments, the light source may comprise a plurality of light emitters. In some embodiments, the light source may comprise at least three light emitters.

The device further comprises a housing configured to house the light source and the imaging sensor. The housing comprises an aperture through which the light source is configured to emit light and the imaging sensor is configured to detect light reflected from the skin.

The aperture is a numerical aperture having a value in a range from 0.005 to 0.3.

In some embodiments, a distance between the light source and the imaging sensor may be greater than 5 millimeters.

According to a second aspect, there is provided a system for measuring skin texture. The system comprises a device as described above and a processor configured to acquire the image of the skin from the imaging sensor and process the acquired image to measure the skin texture.

According to a third aspect, there is provided a method of operating a device to image skin. The device comprises a light source, an imaging sensor, and a housing configured to house the light source and the imaging sensor.

The method comprises emitting light from the light source to illuminate the skin. The light source has a radiation angle in a range from 5 to 40 degrees. The radiation angle is the angle at which an intensity of the light emitted by the light source is half that of a maximum intensity of the light emitted by the light source. Furthermore, the light source emits light with an angle of incidence, relative to the normal direction to a point of incidence of the emitted light on the skin, in a range from 10 to 30 degrees. The method also comprises detecting, by the imaging sensor, light reflected from the skin and converting, by the imaging sensor, the detected light into an image of the skin.

According to a fourth aspect, there is provided a computer program product comprising a computer readable medium, the computer readable medium having computer readable code embodied therein, the computer readable code being configured such that, on execution by a suitable computer or processor, the computer or processor is caused to operate a device described above to perform the method described above.

According to the aspects and embodiments described above, the limitations of existing devices are addressed. In particular, according to the above-described aspects and embodiments, images of improved contrast (or resolution) (and thus improved quality) can be acquired. The improved contrast in (or resolution of) the images that is achieved is optimum for features of the skin (such as skin texture, fine lines and wrinkles, skin spots, impurities, irregularities, abnormalities, etc.) to be adequately resolved in the images. In this way, the images can be used to provide more accurate skin monitoring and to more reliably detect skin conditions. There is thus provided an improved device for imaging skin, which overcomes existing problems.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a schematic diagram of a device according to an embodiment;
Fig. 2 is a schematic diagram of a radiation angle range for a light source of a device according to an embodiment;
Figs. 3A-B are example images acquired by an imaging sensor with a light source having different radiation angles;
Figs. 4A-F are example images acquired by an imaging sensor with a light source configured to emit light with different angles of incidence;
Fig. 5 is a schematic diagram of a device according to an example embodiment; and
Fig. 6 is a flow chart illustrating a method of operating a device according to an embodiment.

DETAILED DESCRIPTION OF EMBODIMENTS

As noted above, there is provided an improved device for imaging skin, which overcomes existing problems.

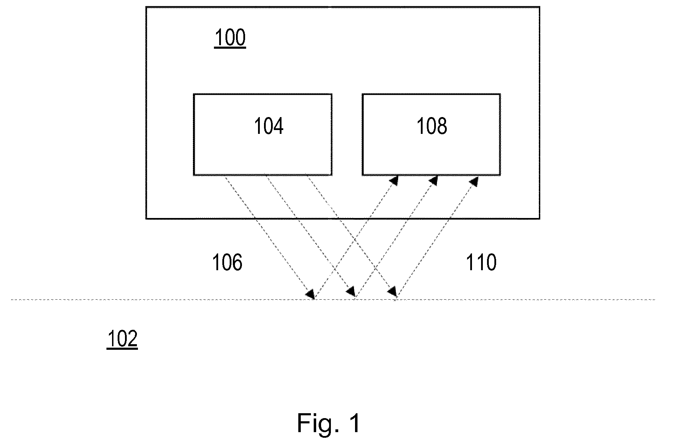

Fig. 1 illustrates a device 100 for imaging skin 102 according to an embodiment. As illustrated in Fig. 1, the device 100 comprises a light source 104. The light source 104 is configured to emit light 106 to illuminate the skin 102. In some embodiments, the light source 104 can, for example, comprise a semiconductor light source such as a light emitting diode (LED), a resonant cavity light emitting diode (RCLED), a vertical cavity laser diode (VCSELs), an edge emitting laser, or any other semiconductor light source. Alternatively or in addition, in some embodiments, the light source 104 can, for example, comprise an organic light source such as a passive-matrix (PMOLED), an active-matrix (AMOLED), or any other organic light source. Alternatively or in addition, in some embodiments, the light source 104 can, for example, comprise a solid state light source. However, although examples for the type of light source 104 have been provided, it will be understood that other types of light source may be used.

As also illustrated in Fig. 1, the device 100 further comprises an imaging sensor 108. The imaging sensor 108 is configured to detect light 110 reflected from the skin 102 and convert the detected light 110 into an image of the skin 102. In some embodiments, the imaging sensor 108 described herein may be, for example, a camera sensor. In some embodiments, the imaging sensor 108 may comprise a focusing lens. Alternatively or in addition, in some embodiments, the imaging sensor 108 may comprise an aperture. In some embodiments, the aperture of the imaging sensor 108 may have a stop size in a range from 0.2 mm to 0.6 mm. Although examples have been provided for the imaging sensor 108, it will be understood that any other type of imaging sensor is also possible.

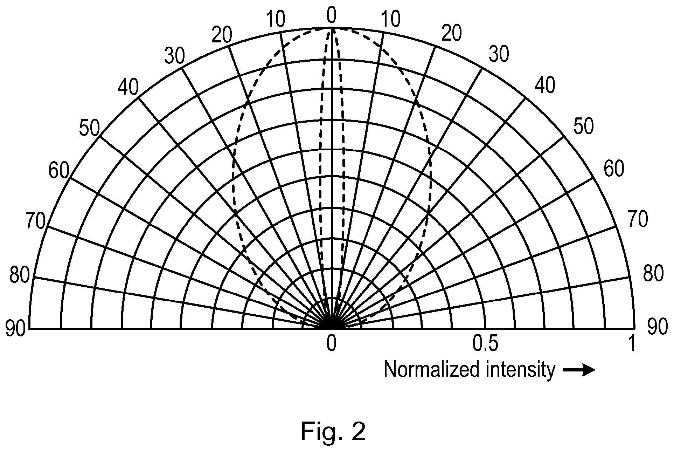

Fig. 2 illustrates a radiation angle range for the light source 104 of the device 100 described herein. The radiation angle referred to herein is the angle at which an intensity (e.g. a luminous intensity) of the light emitted by the light source 104 is half that (i.e. 50%) of a maximum (e.g. peak) intensity of the light emitted by the light source 104. A radiation angle is, for example, a measure of a dispersion (or radiation pattern) of light emitted by a light source. A radiation pattern of a light source viewed on-axis in a narrower viewing angle has a higher intensity than a radiation pattern of a light source viewed on-axis in a wider viewing angle.

As illustrated in Fig. 2, the light source 104 of the device 100 described herein has a radiation angle in a range from 5 to 40 degrees. In some embodiments, for example, the light source 104 of the device 100 can have a radiation angle in a range from 10 to 35 degrees, for example in a range from 15 to 30 degrees, for example in a range from 20 to 25 degrees. In some embodiments, for example, the light source 104 of the device 100 can have a radiation angle in a range from 10 to 30 degrees, for example in a range from 10 to 25 degrees. For example, in some embodiments, the light source 104 of the device 100 can have a radiation angle selected from 10 degrees, 11 degrees, 12 degrees, 13 degrees, 14 degrees, 15 degrees, 16 degrees, 17 degrees, 18 degrees, 19 degrees, 20 degrees, 21 degrees, 22 degrees, 23 degrees, 24 degrees, and 25 degrees, or any non-integer radiation angle between the mentioned values, or any other radiation angle that is in a range from 5 to 40 degrees. Although some examples have been provided for the radiation angle range and the radiation angle for the light source 104 of the device 100, it will be understood that the light source 104 of the device 100 may have any radiation angle in the range from 5 to 40 degrees.

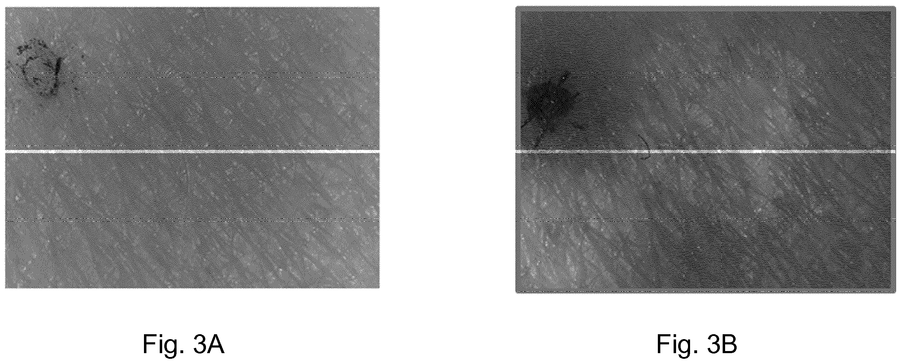

With the light source 104 of the device 100 having the above-described radiation angle, the image of the skin 102 that is acquired from the imaging sensor 108 of the device 100 has an improved contrast (or resolution). In this way, features of the skin (such as skin texture, fine lines and wrinkles, skin spots, impurities, irregularities, abnormalities, or any other features of the skin) can be more adequately resolved and thus better visualized. This is illustrated in Figs. 3A and 3B.

Fig. 3A is an example of an image acquired by an imaging sensor of a device when a light source of the device has a radiation angle of 120 degrees, which falls outside the radiation angle range for the light source 104 of the device 100 described herein. When a light source having a large radiation angle (e.g. any angle above 40 degrees, such as 120 degrees) is used to illuminate an irregular surface such as textured skin, the rays of light emitted by the light source have a broad range of angle of incidence on the surface in the case of diffuse illumination and are therefore less sensitive to any irregularities on the surface. As such, the irregularities are difficult to visualize in an image acquired from an imaging sensor when skin is illuminated with such a light source. By achieving a broad range of angle of incidence for illuminating a textured surface with a large radiation angle (e.g. any angle above 40 degrees, such as 120 degrees), the probability of detecting light reflected from the skin is enhanced, but the sensitivity of the reflected light to any irregularities on the surface of the skin is reduced, which results in an image of poor contrast.

Fig. 3B is an example of an image acquired by an imaging sensor 108 of the device 100 described herein when the light source 104 of the device 100 has a radiation angle of 25 degrees. When the light source 104 of the device 100 described herein having a radiation angle in the range from 5 to 40 degrees is used to illuminate an irregular surface (such as textured skin), the rays of light emitted by the light source have a narrow range of angle of incidence on the surface and are therefore more sensitive to any irregularities on the surface. As such, the irregularities are easier to visualize in an image acquired by the imaging sensor 108 of the device 100 described herein when the skin is illuminated with such a light source 104. Thus, as illustrated in Figs. 3A and 3B, the image acquired with the light source 104 having a radiation angle of 25 degrees has a higher contrast (or resolution) than the image acquired with the light source having a radiation angle of 120 degrees, such that the features in the image are more clearly resolved.

The light source 104 of the device 100 described herein is configured to emit light 106 with an angle of incidence (AOI) in a particular range. The angle of incidence referred to herein is the angle that the light 106 emitted from the light source 104 (that is, the light that is incident on the skin 102 or that illuminates the skin 102) makes with the normal at a point of incidence on the skin 102.

The light source 104 is configured to emit light 106 with an angle of incidence in a range from 10 to 30 degrees, for example in a range from 10 to 25 degrees. For example, in some embodiments, the light source 104 can be configured to emit light 106 with an angle of incidence selected from 10 degrees, 11 degrees, 12 degrees, 13 degrees, 14 degrees, 15 degrees, 16 degrees, 17 degrees, 18 degrees, 19 degrees, 20 degrees, 21 degrees, 22 degrees, 23 degrees, 24 degrees, and 25 degrees, or any non-integer angle of incidence between the mentioned values.

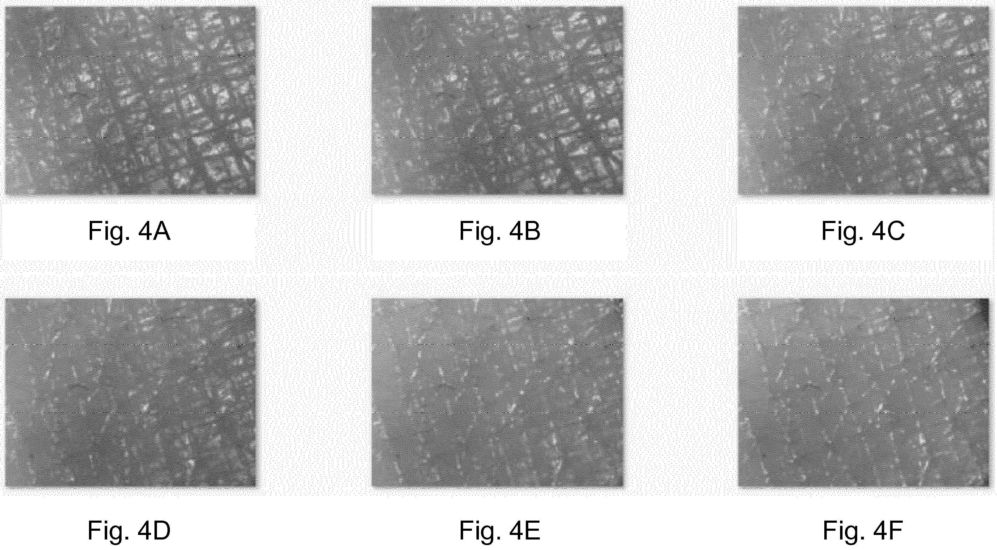

Fig. 4A is an example of an image acquired by the imaging sensor 108 of the device 100 when the light source 104 of the device 100 is configured to emit light 106 with an angle of incidence of 15 degrees. Fig. 4B is an example of an image acquired by the imaging sensor 108 of the device 100 when the light source 104 of the device 100 is configured to emit light 106 with an angle of incidence of 20 degrees. Fig. 4C is an example of an image acquired by the imaging sensor 108 of the device 100 when the light source 104 of the device 100 is configured to emit light 106 with an angle of incidence of 30 degrees. Fig. 4D is an example of an image acquired by the imaging sensor 108 of the device 100 when the light source 104 of the device 100 is configured to emit light 106 with an angle of incidence of 40 degrees. Fig. 4E is an example of an image acquired by the imaging sensor 108 of the device 100 when the light source 104 of the device 100 is configured to emit light 106 with an angle of incidence of 50 degrees. Fig. 4F is an example of an image acquired by an imaging sensor of a device when a light source of the device is configured to emit light with an angle of incidence of 60 degrees.

As illustrated in Figs. 4A-F, the images acquired by the imaging sensor 108 of the device 100 when the light source 104 of the device 100 is configured to emit light 106 with an angle of incidence in the range from 5 to 50 degrees (as illustrated in Figs. 4A-E) have a higher contrast (or resolution) than the image acquired by an imaging sensor of a device when a light source of the device is configured to emit light outside this range, specifically with an angle of incidence of 60 degrees (as illustrated in Fig. 4F). Thus, an angle of incidence in the range from 5 to 50 degrees is optimal for resolving features of the skin (such as skin texture, fine lines and wrinkles, skin spots, impurities, irregularities, abnormalities, or any other features of the skin), whereas at a larger angle of incidence such features of the skin may not be visible in acquired images. As also illustrated in Figs. 4A-F, an angle of incidence in a range from 5 to 40 degrees can provide a further increase in contrast in (or resolution of) the image and can thus provide further improvements for resolving features of the skin.

In some embodiments, the light source 104 may be configured to emit light 106 to uniformly (or homogeneously) illuminate the skin 102. For example, in some embodiments, the light source 104 may be configured to emit light 106 to provide a uniform (or homogenous) light intensity distribution toward the skin 102. More specifically, for example, the light source 104 may be configured to emit light 106 to uniformly illuminate the skin 102 in the field of view of the imaging sensor 108 or to provide a uniform intensity distribution of light toward the skin 102 in the field of view of the imaging sensor 108.

In some embodiments, the light source 104 may comprise a plurality of light emitters suitably arranged to provide uniform illumination to the skin 102. For example, the plurality of light emitters may be provided in a ring or tiled arrangement to provide uniform illumination to the skin 102. Alternatively or in addition, the plurality of light emitters may be tilted to provide uniform illumination to the skin 102. In some examples, the device 100 may comprise a lens (e.g. a Fresnel field lens) configured with light source 104 to provide uniform illumination to the skin 102. While it has been described that the light source 104 may be configured to emit light 106 to uniformly illuminate the skin 102, it will be appreciated that in some embodiments, the light source 104 may be configured to emit light 106 to substantially uniformly illuminate the skin 102 or to emit light 106 to (e.g. substantially) uniformly illuminate an area of skin that is of a predefined size. Although some examples have been provided for the manner in which the light source 104 may be configured to emit light 106 to uniformly illuminate the skin 102, a person skilled in the art will also be aware of other examples. Thus, according to some embodiments, the illumination conditions can prevent non-linear effects or non-uniformities for intensity being present in the field of view.

Thus, as described earlier, the light source 104 of the device 100 described herein has a radiation angle in a range from 5 to 40 degrees and is configured to emit light 106 with an angle of incidence in a range from 10 to 30 degrees. In addition, according to some embodiments, the light source 104 may be configured or further configured to emit light 106 to uniformly illuminate the skin 102. In this way, images acquired by the imaging sensor 108 of the device 100 have an improved contrast (or resolution), such that it is possible to optimally resolve features of the skin (such as skin texture, fine lines and wrinkles, skin spots, impurities, irregularities, abnormalities, or any other features of the skin) in the acquired images. There is thus described herein a device 100 that provides optimal illumination conditions for imaging skin.

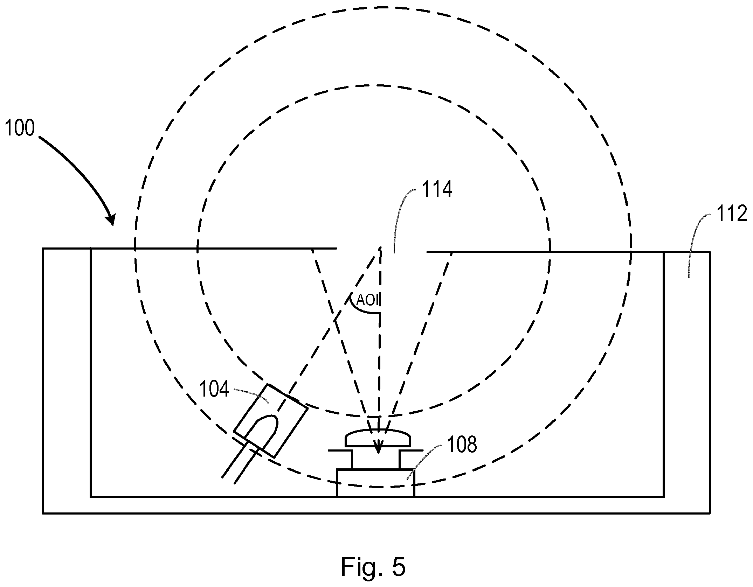

Fig. 5 illustrates the device 100 for imaging skin 102 according to an example embodiment. The device 100 according to this illustrated example embodiment is as described earlier with reference to Fig. 1. In particular, the device 100 comprises a light source 104 configured to emit light 106 to illuminate the skin 102 and an imaging sensor 108 configured to detect light 110 reflected from the skin 102 and convert the detected light 110 into an image of the skin 102. As described earlier with reference to Fig. 2, the light source 104 of the device 100 has a radiation angle in a range from 5 to 40 degrees. As described earlier, the light source 104 of the device 100 is configured to emit light 106 with an angle of incidence in a range from 10 to 30 degrees. In some embodiments, as also described earlier, the light source 104 may be configured to emit light 106 to uniformly illuminate the skin 102.

In the embodiment illustrated in Fig. 5, the device 100 further comprises a housing 112 configured to house the light source 104 and the imaging sensor 108. The housing 112 comprises an aperture 114 through which the light source 104 is configured to emit light 106 and the imaging sensor 108 is configured to detect light 110 reflected from the skin 102. In some embodiments, the device 100 can comprise a numerical aperture, e.g. the aperture 114 can be a numerical aperture. Therefore, in some embodiments, a numerical aperture value may be associated with the device 100. In some embodiments, the device 100 may comprise a lens, which may define the numerical aperture value of device 100. In some embodiments, a numerical aperture value may be determined according to the following equation:

$$NA_i = n sin\theta, \qquad (1)$$

where $NA_i$ is the numerical aperture value, n is the refractive index of the medium (e.g. air) in which the device 100 is used, and $\theta$ is the maximal half-angle of the cone of light that can enter or exit the aperture.

The numerical aperture value may also be determined according to the following equation:

$$NA_i = nsin\theta = nsin\left[arctan\left(\frac{D}{2f}\right)\right] \approx n\frac{D}{2f}, \qquad (2)$$

where D is the stop size of the aperture and f is the focal length (or distance to the skin 102). Therefore, from equation (2) and assuming the device 100 is used in air (with n=1), the numerical aperture value may be related to the f-number (N) of the device 100 by the following equation:

$$N = \frac{1}{2NA_i}. \qquad (3)$$

In some of embodiments involving a numerical aperture, the numerical aperture may have a value in a range from 0.005 to 0.3. In some embodiments, for example, the numerical aperture may have a value in a range from 0.01 to 0.275, for example in a range from 0.015 to 0.25, for example in a range from 0.02 to 0.225, for example in a range from 0.025 to 0.2, for example in a range from 0.03 to 0.175, for example in a range from 0.035 to 0.15, for example in a range from 0.04 to 0.125, for example in a range from 0.045 to 0.1, for example in a range from 0.05 to 0.075. Although some examples have been provided for the numerical aperture value range and the numerical aperture value of the device 100 (e.g. of the housing 112 of the device 100), it will be understood that the numerical aperture of the device (e.g. of the housing 112 of the device 100) may be any value in the range from 0.005 to 0.3.

In some embodiments, the light source 104 described herein may be configured to emit unpolarized light. In some of these embodiments, the device 100 may further comprise a polarizer (not illustrated). The polarizer can be configured to polarize the light 110 reflected from the skin 102. Thus, the polarizer may be positioned such that light 110 reflected from the skin 102 reaches the polarizer before the imaging sensor 108. For example, the polarizer can be positioned before (e.g. in front of) the imaging sensor 108. In embodiments where the device 100 comprises a polarizer, the imaging sensor 108 can be configured to detect the polarized light 110 reflected from the skin 102. Alternatively, in other embodiments, the light source 104 described herein may be configured to emit polarized light. In these embodiments, the imaging sensor 108 can be configured to detect the polarized light 110 reflected from the skin 102.

In some embodiments, the light source 104 described herein may comprise a single light emitter. In other embodiments, the light source 104 described herein may comprise a plurality of light emitters. For example, according to some embodiments, the light source 104 described herein may comprise at least two light emitters, at least three light emitters, at least four light emitters, at least five light emitters, at least six light emitters, or any other number of light emitters. In embodiments where the light source 104 comprises a plurality of light emitters, the plurality of light emitters may be configured together as a single lighting module. In some embodiments, the light source 104 (and thus the one or more light emitters) may be provided (or mounted) on a substrate, such as a semiconductor substrate or a printed circuit board (PCB). Thus, according to some embodiments, the light source 104 may be a chip-on-board (COB) light source. In some embodiments where the light source 104 comprises a plurality of light emitters, the plurality of light emitters may be provided (or mounted) on the same substrate.

The plurality of light emitters may comprise any type of light emitter or any combination of types of light emitter, such as any of the types provided earlier in respect of the light source 104 of the device 100. For example, the plurality of light emitters may comprise any one or more of a semiconductor light emitter (such as a light emitting diode LED, a resonant cavity light emitting diode RCLED, a vertical cavity laser diode VCSEL, an edge emitting laser, or any other semiconductor light emitter, or any combination of semiconductor light emitter), an organic light emitter (such as a passive-matrix PMOLED, an active-matrix AMOLED, or any other organic light emitter, or any combination of organic light emitters), a solid state light emitter, or any other light emitter, or any combination of light emitters.

In some embodiments, the light source 104 described herein may be configured to emit visible light, such as white light. In some embodiments where the light source 104 is configured to emit visible light, the visible light may have a wavelength in a range from 389 nm to 780 nm. In some embodiments where the light source 104 is configured to emit white light, the white light may have a correlated color temperature (CCT) in a range from 2000 K to 20000 K, for example in a range from 2700 K to 20000 K, for example in a range from 2700 K to 6500 K. Alternatively or in addition to visible light, in some embodiments, the light source 104 described herein may be configured to emit infrared (IR) light, such as near infrared (NIR) light. In some embodiments where the light source 104 is configured to emit infrared light, the infrared light may have a wavelength in a range from 750 nm to 3000 nm. Although some examples have been provided for the type of light source 104, it will be appreciated that the device 100 may alternatively or in addition comprise any other type of light source or light sources suitable to illuminate the skin 102.

In some embodiments, a distance between the light source 104 and the imaging sensor 108 of the device 100 described herein may be greater than 5 millimeters (mm). In some embodiments, a distance between the light source 104 and the imaging sensor 108 of the device 100 described herein may be in a range from 6 mm to 14 mm, for example in a range from 7 mm to 13 mm, for example in a range from 8 mm to 12 mm, for example in a range from 9 mm to 11 mm. For example, in some embodiments, the distance between the light source 104 and the imaging sensor 108 may be a distance selected from 6 mm, 7 mm, 8 mm, 9 mm, 10 mm, 11 mm, 12 mm, 13 mm and 14 mm, or any non-integer distance between the mentioned values, or any other distance greater than 5 millimeters. In any of embodiments described herein, the imaging sensor 108 of the device 100 described herein may have a particular depth of focus (DOF). For example, in some embodiments, the imaging sensor 108 may have a depth of focus (DOF) that is greater than 2 mm.

Although not illustrated, there is also provided a system for measuring skin texture. The system comprises a device 100 according to any of the embodiments described herein. The system also comprises a processor. The processor is configured to acquire the image of the skin 102 from the imaging sensor 108 and process the acquired image to measure the skin texture. By implementing the device 100 according to any of the embodiments described herein in the system, optimal illumination conditions (or settings) can be provided for resolving skin texture. Thus, an improved system for measuring skin texture is provided.

In some embodiments, the processor may be configured to detect a color contrast in the acquired image and measure the skin texture by determining (or deriving) a two-dimensional texture parameter from the detected color contrast in the acquired image. In embodiments where the device 100 comprises a housing 112 configured to house the light source 104 and the imaging sensor 108 and the housing 112 comprises an aperture 114 (such as in the example embodiment illustrated in Fig. 5), the color contrast in the acquired image.

In some embodiments, the device 100 itself may comprise the processor. In other embodiments, the processor may be external to (i.e. separate to or remote from) the device 100. For example, in some embodiments, the processor may be a separate entity or part of another device.

The processor can be implemented in numerous ways, with software and/or hardware, to perform the various functions described herein. In particular implementations, the processor can comprise a plurality of software and/or hardware modules, each configured to perform, or that are for performing, individual or multiple steps of the method described herein. The processor may comprise one or more processors (such as one or more microprocessors, one or more multi-core processors and/or one or more digital signal processors (DSPs)), one or more processing units, and/or one or more controllers (such as one or more **microcontrollers)** that may be configured or programmed (e.g. using software or computer program code) to perform the various functions described herein. The processor may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, controllers, DSPs and associated circuitry) to perform other functions.

Although the device 100 has been described herein with reference to Figs. 1 and 5, it will be appreciated that Figs. 1 and 5 only show the components required to illustrate certain embodiments and, in a practical implementation, the device 100 may comprise other components or additional components to those shown.

For example, although not illustrated in Figs. 1 or 5, the device 100 may comprise a communications interface (or communications circuitry). The communications interface can be for enabling the device 100 or components of the device 100 to communicate with and/or connect to one or more other devices, components, interfaces, memories, or sensors (such as any of those described herein). For example, the communications interface can be for enabling the imaging sensor 108 of the device 100 to communicate with and/or connect to the processor described earlier for image processing. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications.

Although also not illustrated in Figs. 1 or 5, the device 100 may comprise a memory, or may be configured to communicate with and/or connect to a memory external to (i.e. separate to or remote from) the device 100. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). The memory can be configured to store program code that can be executed by a processor to cause the device 100 to operate in the manner described herein. Alternatively or in addition, the memory can be configured to store information acquired by the device 100. For example, in some embodiments, the memory may be configured to store an image of skin acquired by the imaging sensor 108 of the device 100, a skin texture measured by the processor of the system, or any other information, or any combination of information resulting from the method described herein.

Although also not illustrated in Figs. 1 or 5, the device 100 may comprise a user interface, or may be configured to communicate with and/or connect to a user interface external to (i.e. separate to or remote from) the device 100. The user interface can be configured to render (or output, display, or provide) information resulting from the method described herein. For example, in some embodiments, the user interface may be configured to render (or output, display, or provide) an image of skin acquired by the imaging sensor 108, a skin texture measured by the processor, or any other information, or any combination of information resulting from the method described herein. Alternatively or in addition, the user interface can be configured to receive a user input. For example, the user interface may allow a user to manually enter information or instructions, interact with and/or control the device 100. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. **In** some embodiments, the user interface that is controlled to render information may be the same user interface as that which enables the user to provide a user input.

Although also not illustrated in Figs. 1 or 5, the device 100 may comprise a battery or other power supply for powering the device 100 or means for connecting the device 100 to a mains power supply, or any other component, or any combination of components.

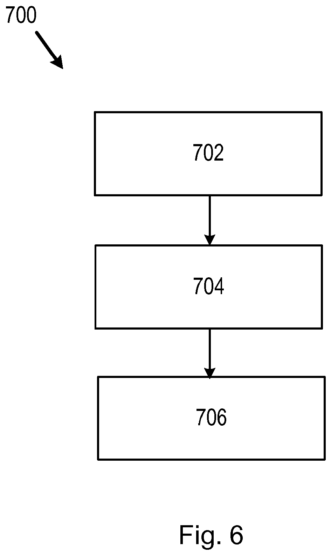

Fig. 6 illustrates a method 700 of operating the device 100 described herein to image skin 102 according to an embodiment. **In** some embodiments, the method 700 may generally be performed by or under the control of a processor, such as the processor mentioned earlier. At block 702, light is emitted from the light source 104 of the device 100 to illuminate the skin 102. At block 704, light reflected from the skin is detected by the imaging sensor 108 of the device 100. At block 706, the detected light is converted by the imaging sensor 108 into an image of the skin. Although not illustrated in Fig. 6, according to some embodiments, the method 700 may further comprise a processor processing the acquired image to measure skin texture, as described earlier.

There is also provided a computer program product comprising a computer readable medium. The computer readable medium has computer readable code embodied therein. The computer readable code is configured such that, on execution by a suitable computer or processor, the computer or processor is caused to operate the device described herein to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved device 100 for imaging skin 102, a method 700 of operating the device 100 to image skin 102, an improved system for measuring skin texture, a method of operating the system to measure skin texture, and a computer program product. According to these aspects, images of improved contrast (or resolution) (and thus improved quality) can be acquired. The improved contrast in (or resolution of) the images achieved by the device 100 described herein is optimum for adequately resolving features of the skin (such as skin texture, fine lines and wrinkles, skin spots, impurities, irregularities, abnormalities, or any other features of the skin). For example, it can be possible to achieve a resolution of more than 20 lines per millimeter (lnp/mm), for example a resolution of more than 25 lnp/mm. In effect, an optimal parameter space for resolving skin texture is provided. This is possible through the use of a small radiation angle light source 104 (e.g. with maximum intensity distribution on the optical axis) and also with one or more of the light source 104 having a small angle of incidence and optionally being configured to provide a uniform light intensity distribution (e.g. in a narrow range of angles in the field of view). Thus, according to the aspects and embodiments described herein, images acquired by the imaging sensor 108 of the device 100 can be used to provide more accurate skin monitoring and to more reliably detect skin conditions.

Variations to the disclosed embodiments can be understood and effected by those skilled in the art in practicing the principles and techniques described herein, from a study of the drawings, the disclosure and the appended claims. **In** the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A device (100) for imaging skin (102), the device (100) comprising:

- a light source (104) configured to emit light (106) to illuminate the skin (102),
- an imaging sensor (108) configured to detect light (110) reflected from the skin (102) and convert the detected light (110) into an image of the skin (102), wherein the imaging sensor is located at a normal position relative to a point of incidence of the emitted light on the skin; and
- a housing (112) configured to house the light source (104) and the imaging sensor (108) and comprising an aperture (114) through which the light source (104) is configured to emit light (106) and the imaging sensor (108) is configured to detect light (110) reflected from the skin (102);
wherein the light source (104) has a radiation angle in a range from 5 to 40 degrees, wherein the radiation angle is the angle at which an intensity of the light emitted by the light source (104) is half that of a maximum intensity of the light (106) emitted by the light source (104); and
wherein the light source (104) is configured to emit light (106) with an angle of incidence, relative to the normal direction to the point of incidence of the emitted light on the skin, in a range from 10 to 30 degrees.

2. A device (100) as claimed in claim 1, wherein the light source (104) has a radiation angle in a range from 10 to 30 degrees.

3. A device (100) as claimed in claim 2, wherein the light source (104) has a radiation angle in a range from 10 to 25 degrees.

4. A device (100) as claimed in any one of the preceding claims, wherein the light source (104) is configured to emit light (106) with an angle of incidence in a range from 10 to 25 degrees.

5. A device (100) as claimed in any one of the preceding claims, wherein the light source (104) comprises a plurality of light emitters, the plurality of light emitters being configured to emit light (106) to uniformly illuminate the skin.

6. A device (100) as claimed in claim 5, wherein the light source (104) comprises at least three light emitters.

7. A device (100) as claimed in any one of claims 1 to 4, wherein the device (100) further comprises a lens, particularly a Fresnel field lens, configured with light source (104) to provide uniform illumination to the skin (102).

8. A device (100) as claimed in any one of the preceding claims, wherein a distance between the light source (104) and the imaging sensor (106) is greater than 5 millimeters.

9. A device (100) as claimed in any one of the preceding claims, wherein the device comprises a numerical aperture having a value in the range from 0.005 to 0.3.

10. A system for measuring skin texture, the system comprising:

a device (100) as claimed in any one of the preceding claims; and
a processor configured to acquire the image of the skin (102) from the imaging sensor (108) and process the acquired image to measure the skin texture.

11. A method (700) of operating a device (100) to image skin (102), wherein the device (100) comprises a light source (104), an imaging sensor (108), and
a housing (112) configured to house the light source (104) and the imaging sensor (108), the housing comprising an aperture through which the light source (104) is configured to emit light (106) and the imaging sensor (108) is configured to detect light (110) reflected from the skin (102), and the method (700) comprises:

emitting (702) light (106) from the light source (104) to illuminate the skin (102), wherein the light source (104) has a radiation angle in a range from 5 to 40 degrees, wherein the radiation angle is the angle at which an intensity of the light (106) emitted by the light source (104) is half that of a maximum intensity of the light (106) emitted by the light source (104); and wherein the light source (104) is configured to emit light (106) with an angle of incidence, relative to the normal direction to a point of incidence of the emitted light on the skin, in a range from 10 to 30 degrees;
detecting (704), by the imaging sensor (108), light (110) reflected from the skin (102), wherein the imaging sensor is located at a normal position relative to the point of incidence of the emitted light on the skin; and
converting (706), by the imaging sensor (108), the detected light (110) into an image of the skin (102).

12. A computer program product comprising a computer readable medium, the computer readable medium having computer readable code embodied therein, the computer readable code being configured such that, on execution by a suitable computer or processor, the computer or processor is caused to operate a device (100) according to claims 1-9 to image skin (102) according to the method for operating such a device (100) as claimed in claim 11.

**Patentansprüche**

1. Vorrichtung (100) zur Bildgebung der Haut (102), wobei die Vorrichtung (100) umfasst:

    - eine Lichtquelle (104), die konfiguriert ist, Licht (106) zu emittieren, um die Haut (102) zu beleuchten,
    - einen Bildsensor (108), der konfiguriert ist, von der Haut (102) reflektiertes Licht (110) zu erfassen und das erfasste Licht (110) in ein Bild der Haut (102) umzuwandeln, wobei sich der Bildsensor an einer normalen Position in Bezug zu einer Einfallspunkt des emittierten Lichts auf der Haut befindet; und
    - ein Gehäuse (112), das zur Aufnahme der Lichtquelle (104) und des Bildsensors (108) konfiguriert ist und eine Öffnung (114) umfasst, durch die die Lichtquelle (104) konfiguriert ist, Licht (106) zu emittieren, und der Bildsensor (108) konfiguriert ist, das von der Haut (102) reflektierte Licht (110) zu erfassen;
    wobei die Lichtquelle (104) einen Abstrahlwinkel im Bereich von 5 bis 40 Grad aufweist, wobei der Abstrahlwinkel der Winkel ist, bei dem die Intensität des von der Lichtquelle (104) emittierten Lichts die Hälfte der maximalen Intensität des von der Lichtquelle (104) emittierten Lichts (106) beträgt; und
    wobei die Lichtquelle (104) konfiguriert ist, Licht (106) mit einem Einfallswinkel in Bezug zur Normalenrichtung zum Einfallspunkt des emittierten Lichts auf die Haut zu emittieren, der im Bereich von 10 bis 30 Grad liegt.

2. Vorrichtung (100) nach Anspruch 1, wobei die Lichtquelle (104) einen Abstrahlwinkel im Bereich von 10 bis 30 Grad aufweist.

3. Vorrichtung (100) nach Anspruch 2, wobei die Lichtquelle (104) einen Abstrahlwinkel im Bereich von 10 bis 25 Grad aufweist.

4. Vorrichtung (100) nach einem der vorstehenden Ansprüche, wobei die Lichtquelle (104) konfiguriert ist, Licht (106) mit einem Einfallswinkel im Bereich von 10 bis 25 Grad zu emittieren.

5. Vorrichtung (100) nach einem der vorstehenden Ansprüche, wobei die Lichtquelle (104) eine Vielzahl von Lichtemittern umfasst, die konfiguriert sind, Licht (106) zu emittieren, um die Haut gleichmäßig zu beleuchten.

6. Vorrichtung (100) nach Anspruch 5, wobei die Lichtquelle (104) mindestens drei Lichtemitter umfasst.

7. Vorrichtung (100) nach einem der Ansprüche 1 bis 4, wobei die Vorrichtung (100) weiter eine Linse umfasst, insbesondere eine Fresnel-Feldlinse, die mit einer Lichtquelle (104) konfiguriert ist, eine gleichmäßige Beleuchtung der Haut (102) bereitzustellen.

8. Vorrichtung (100) nach einem der vorstehenden Ansprüche, wobei der Abstand zwischen der Lichtquelle (104) und dem Bildsensor (106) größer als 5 Millimeter ist.

9. Vorrichtung (100) nach einem der vorstehenden Ansprüche, wobei die Vorrichtung eine numerische Öffnung umfasst, die einen Wert im Bereich von 0,005 bis 0,3 aufweist.

10. System zum Messen der Hauttextur, das System umfassend:

    eine Vorrichtung (100) nach einem der vorstehenden Ansprüche; und
    einen Prozessor, der konfiguriert ist, das Bild der Haut (102) vom Bildsensor (108) zu erlangen und das erlangte Bild zu verarbeiten, um die Hauttextur zu messen.

11. Verfahren (700) zum Betreiben einer Vorrichtung (100) zur Bildgebung der Haut (102), wobei die Vorrichtung (100) eine Lichtquelle (104), einen Bildsensor (108) und ein Gehäuse (112) umfasst, das zur Aufnahme der Lichtquelle (104) und des Bildsensors (108) konfiguriert ist, wobei das Gehäuse eine Öffnung umfasst, durch die die Lichtquelle (104) konfiguriert ist, Licht (106) zu emittieren und der Bildsensor (108) konfiguriert ist, das von der Haut (102) reflektierte Licht (110) zu erfassen, und das Verfahren (700) umfassend:

Emittieren (702) von Licht (106) von der Lichtquelle (104) zur Beleuchtung der Haut (102), wobei die Lichtquelle (104) einen Abstrahlwinkel im Bereich von 5 bis 40 Grad aufweist, wobei der Abstrahlwinkel der Winkel ist, bei dem die Intensität des von der Lichtquelle (104) emittierten Lichts (106) die Hälfte der maximalen Intensität des von der Lichtquelle (104) emittierten Lichts (106) beträgt; und wobei die Lichtquelle (104) konfiguriert ist, Licht (106) mit einem Einfallswinkel in Bezug zur Normalenrichtung auf einen Einfallspunkt des emittierten Lichts auf der Haut im Bereich von 10 bis 30 Grad zu emittieren;

Erfassen (704) des von der Haut (102) reflektierten Lichts (110) durch den Bildsensor (108), wobei sich der Bildsensor an einer normalen Position in Bezug zum Einfallspunkt des emittierten Lichts auf der Haut befindet; und

Umwandeln (706) des erfassten Lichts (110) in ein Bild der Haut (102) durch den Bildsensor (108).

**12.** Computerprogrammprodukt, das ein computerlesbares Medium umfasst, wobei das computerlesbare Medium einen darin verkörperten computerlesbaren Code aufweist, wobei der computerlesbare Code so konfiguriert ist, dass bei Ausführung durch einen geeigneten Computer oder Prozessor der Computer oder Prozessor dazu veranlasst wird, eine Vorrichtung (100) nach den Ansprüchen 1-9 zur Bildgebung der Haut (102) nach dem Verfahren zum Betreiben einer solchen Vorrichtung (100) nach Anspruch 11 zu betreiben.

**Revendications**

**1.** Dispositif (100) pour imager de la peau (102), le dispositif (100) comprenant :

- une source de lumière (104) configurée pour émettre de la lumière (106) pour éclairer la peau (102),
- un capteur d'imagerie (108) configuré pour détecter la lumière (110) réfléchie par la peau (102) et convertir la lumière détectée (110) en une image de la peau (102), dans lequel le capteur d'imagerie est situé à une position normale par rapport à un point d'incidence de la lumière émise sur la peau ; et
- un boîtier (112) configuré pour loger la source de lumière (104) et le capteur d'imagerie (108) et comprenant un orifice (114) par lequel la source de lumière (104) est configurée pour émettre de la lumière (106) et le capteur d'imagerie (108) est configuré pour détecter de la lumière (110) réfléchie par la peau (102) ;

dans lequel la source de lumière (104) présente un angle de rayonnement dans une plage allant de 5 à 40 degrés, dans lequel l'angle de rayonnement est l'angle pour lequel une intensité de la lumière émise par la source de lumière (104) est égale à la moitié de l'intensité maximale de la lumière (106) émise par la source de lumière (104) ; et

dans lequel la source de lumière (104) est configurée pour émettre de la lumière (106) avec un angle d'incidence, par rapport à la direction normale du point d'incidence de la lumière émise sur la peau, dans une plage allant de 10 à 30 degrés.

**2.** Dispositif (100) selon la revendication 1, dans lequel la source de lumière (104) présente un angle de rayonnement dans une plage allant de 10 à 30 degrés.

**3.** Dispositif (100) selon la revendication 2, dans lequel la source de lumière (104) présente un angle de rayonnement dans une plage allant de 10 à 25 degrés.

**4.** Dispositif (100) selon l'une quelconque des revendications précédentes, dans lequel la source de lumière (104) est configurée pour émettre de la lumière (106) avec un angle d'incidence dans une plage allant de 10 à 25 degrés.

**5.** Dispositif (100) selon l'une quelconque des revendications précédentes, dans lequel la source de lumière (104) comprend une pluralité d'émetteurs de lumière, la pluralité d'émetteurs de lumière étant configurée pour émettre de la lumière (106) pour éclairer uniformément la peau.

**6.** Dispositif (100) selon la revendication 5, dans lequel la source de lumière (104) comprend au moins trois émetteurs de lumière.

**7.** Dispositif (100) selon l'une quelconque des revendications 1 à 4, dans lequel le dispositif (100) comprend en outre une lentille, notamment une lentille de champ de Fresnel, configurée avec une source de lumière (104) pour fournir un éclairage uniforme à la peau (102).

**8.** Dispositif (100) selon l'une quelconque des revendications précédentes, dans lequel une distance entre la source de lumière (104) et le capteur d'imagerie (106) est supérieure à 5 millimètres.

9. Dispositif (100) selon l'une quelconque des revendications précédentes, dans lequel le dispositif comprend une ouverture numérique présentant une valeur dans la plage allant de 0,005 à 0,3.

10. Système pour mesurer la texture de la peau, le système comprenant :

    un dispositif (100) selon l'une quelconque des revendications précédentes ; et
    un processeur configuré pour acquérir l'image de la peau (102) à partir du capteur d'imagerie (108) et traiter l'image acquise pour mesurer la texture de la peau.

11. Procédé (700) de fonctionnement d'un dispositif (100) pour imager de la peau (102), dans lequel le dispositif (100) comprend une source de lumière (104), un capteur d'imagerie (108) et un boîtier (112) configuré pour loger la source de lumière (104) et le capteur d'imagerie (108), le boîtier comprenant un orifice par lequel la source de lumière (104) est configurée pour émettre de la lumière (106) et le capteur d'imagerie (108) est configuré pour détecter de la lumière (110) réfléchie par la peau (102), et le procédé (700) comprend :

    l'émission (702) de la lumière (106) à partir de la source de lumière (104) pour éclairer la peau (102), dans lequel la source de lumière (104) présente un angle de rayonnement dans une plage allant de 5 à 40 degrés, dans lequel l'angle de rayonnement est l'angle auquel une intensité de la lumière (106) émise par la source de lumière (104) est la moitié de l'intensité maximale de la lumière (106) émise par la source de lumière (104) ; et dans lequel la source de lumière (104) est configurée pour émettre de la lumière (106) avec un angle d'incidence, par rapport à la direction normale à un point d'incidence de la lumière émise sur la peau, dans une plage allant de 10 à 30 degrés ; la détection (704), par le capteur d'imagerie (108), de la lumière (110) réfléchie par la peau (102), dans lequel le capteur d'imagerie est situé à une position normale par rapport au point d'incidence de la lumière émise sur la peau ; et
    la conversion (706), par le capteur d'imagerie (108), de la lumière détectée (110) en une image de la peau (102).

12. Produit de programme informatique comprenant un support lisible par ordinateur, le support lisible par ordinateur présentant un code lisible par ordinateur intégré dans celui-ci, le code lisible par ordinateur étant configuré de telle sorte que, lors de son exécution par un ordinateur, ou un processeur, approprié, l'ordinateur, ou le processeur, soit amené à faire fonctionner un dispositif (100) selon les revendications 1-9 pour imager de la peau (102) selon le procédé de fonctionnement d'un tel dispositif (100) selon la revendication 11.

100

104          108

106                    110

102

Fig. 1

Fig. 2

EP 3 731 735 B1

Fig. 3B

Fig. 3A

Fig. 4C

Fig. 4F

Fig. 4B

Fig. 4E

Fig. 4A

Fig. 4D

Fig. 5

EP 3 731 735 B1

700

702

704

706

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013026846 A
- US 20140243685 A1
- US 20150297130 A1